Europäisches Patentamt

European Patent Office

Office européen des brevets

(19)

(11) Veröffentlichungsnummer: **0 313 718 B1**

# (12) EUROPÄISCHE PATENTSCHRIFT

(45) Veröffentlichungstag der Patentschrift: **09.12.92**

(21) Anmeldenummer: **88103576.0**

(22) Anmeldetag: **08.03.88**

(51) Int. Cl.5: **C07G 17/00**, A61K 35/10, C08H 5/00

(54) **Verfahren zur Herstellung einer Huminatfraktion.**

(30) Priorität: **29.10.87 DE 3736623**

(43) Veröffentlichungstag der Anmeldung:
**03.05.89 Patentblatt 89/18**

(45) Bekanntmachung des Hinweises auf die Patenterteilung:
**09.12.92 Patentblatt 92/50**

(84) Benannte Vertragsstaaten:
**AT BE CH DE ES FR GB GR IT LI LU NL SE**

(56) Entgegenhaltungen:
**DE-C- 712 697**

**CHEMICAL ABSTRACTS, Band 100, Nr. 12, 19. März 1984, Seite 161, Zusammenfassung Nr. 88467z, Columbus, Ohio, US; R. WALTER: "Ultrafiltration as a new method for characterizing humic acids in peat", & RECENT TECHNOL. USE PEAT, REP. INT. SYMP. 1979 (Pub. 1983), 63-70**

**CHEMICAL ABSTRACTS, Band 79, Nr. 24, 17. Dezember 1973, Seite 68, Zusammenfassung Nr. 138398y, Columbus, Ohio, US; S.S. DRAGUNOV et al.: "Chemical characteristics and features of the dissolution of peat humic acids", & KHIM. TVERD. TOPL. 1973, (3), 57-62**

(73) Patentinhaber: **RÜTGERSWERKE AKTIENGESELLSCHAFT**
**Mainzer Landstrasse 217**
**W-6000 Frankturt am Main 1(DE)**

(72) Erfinder: **Seubert, Bernhard**
**Meisenweg 15**
**W-6803 Edingen-Neckarhausen 1(DE)**
Erfinder: **Fickert, Werner, Dr.**
**Stockacher Strasse 14**
**W-6800 Mannheim 61(DE)**
Erfinder: **Spitaler, Ulrich, Dr.**
**Dürkheimer Hohl 2**
**W-6713 Freinsheim(DE)**

**Beschreibung**

Die Erfindung betrifft ein neues Verfahren zur Herstellung einer Fraktion von Alkali- oder Ammoniumsalzen von Huminsäuren mit niedrigem Molekulargewicht.

Huminsäuren oder allgemein Huminstoffen, die in Mooren, Torf, Farberden oder in huminstoffhaltigen Wassern vorkommen, werden heilende Wirkungen zugesprochen (vergleiche Ziechmann, Therapiewoche 28 (1978, 1199-1211). Andererseits sind die Huminsäuren bzw. ihre Salze parenteral als toxisch anzusehen (vergleiche Kühnert et al., Arch. exper. Vet. med. 36 (1982), 169-177), weshalb die Huminstoffe wohl, abgesehen von Moorbädern, vorwiegend in der Veterinärmedizin eingesetzt werden.

In einer früheren Anmeldung der Anmelderin (EP-A-281 679) wird eine Fraktion von Alkalihuminaten beansprucht, deren parenterale Toxität wesentlich geringer ist als die der beschriebenen Huminate, und die eine gute heilende Wirkung besitzt. Die Huminate dieser Fraktion haben eine mittlere relative Molmasse von 1000 g/mol bei einem Streubereich von 300 bis 1500 g/mol.

Diese Alkalihuminatfraktion wird hergestellt, indem huminstoffhaltige Produkte in alkalischer oder ammoniakalischer wäßriger Lösung aufgeschlämmt werden, die entstehende Suspension von groben Feststoffen abgetrennt und mittels Zentrifugieren von feinsten Feststoffen und hochmolekularen Huminaten befreit, die so erhaltene Lösung neutralisiert und bei einem pH-Wert im Bereich von 6,2 bis 7,2 gepuffert und nochmals durch Zentrifugieren gereinigt und von dieser Lösung eine niedermolekulare Fraktion abgetrennt wird.

Dieses Verfahren ist relativ aufwendig, langwierig und für eine wirtschaftliche Gewinnung der Huminate wenig geeignet. Zudem wird das erhaltene Produkt durch die wiederholte pH-Variierung mit zusätzlichen unerwünschten Salzen belastet. Außerdem können durch die pH-Verschiebungen Änderungen in der Struktur der Huminstoffe und damit Ausbeuteverluste in der niedermolekularen Fraktion auftreten.

Es ist daher Aufgabe der Erfindung, ein einfaches, wirtschaftliches Verfahren zur Herstellung jener nicht toxischen, niedermolekularen Huminate bereitzustellen, bei dem die Huminate möglichst nicht durch zusätzliche Salze belastet sind und bei dem durch pH-Verschiebung bedingte Änderungen der Huminstoffe weitgehend vermieden werden.

Die Lösung der Aufgabe erfolgt durch ein Verfahren zur Herstellung einer niedermolekularen Huminatfraktion gemäß der Ansprüche 1 - 13.

Es wurde gefunden, daß sich die gewünschte niedermolekulare Huminatfraktion in großindustriell wirtschaftlich durchführbarer Weise aus huminstoffhaltigen Produkten gewinnen läßt, wenn diese in Wasser suspendiert und mit einer alkalisch wirkenden Substanz so langsam versetzt wird, daß der pH-Wert 7 nicht überschritten wird.

Nach einer relativ kurzen Nachrührzeit von 30 bis 180 min können die Feststoffe der Suspension sedimentieren und die weitgehend feststofffreie Lösung etwa durch abhebern oder dekantieren von den sedimentierten Produkten abgetrennt werden. Diese Lösung wird danach zentrifugiert und der Ultrafiltration unterworfen. Erhalten wird eine sterile, gebrauchsfertige Wirkstofflösung mit einem Gehalt an Huminaten von 2 - 10 Gew.-%. Diese Lösung ist stabil, d. h., die an sich bei Huminatlösungen beobachteten Veränderungen im Sinne einer Weiterpolymerisation werden auch bei längerer Lagerung bei erhöhten Temperaturen nicht beobachtet.

Ausgangsprodukte für die erfindungsgemäßen Huminate können alle huminstoffhaltigen Produkte sein, wie z. B. Torf, Moor, Braunkohle, Farberden oder huminstoffhaltige Wasser bzw. deren Humusschlamm.

Diese huminstoffhaltigen, bevorzugt huminstoffreichen Substrate mit Gehalten von mindestens 5, maximal etwa 50 % Huminsäuren, werden in Wasser aufgeschlämmt und unter Rühren mit einer alkalisch wirkenden Substanz so vorsichtig versetzt, daß der pH-Wert der Reaktionsmischung im Bereich zwischen 6 und 7 liegt und den Wert 7 keinesfalls übersteigt. Der bevorzugte pH-Bereich ist 6,3 - 6,5.

Als alkalisch wirkende Substanz kann die wäßrige Lösung eines Alkalihydroxids, eines durch Hydrolyse alkalisch reagierenden Salzes eines Alkalimetalls mit einer schwachen Säure, eines Amins oder des Ammoniaks dienen, aber auch direkt in das Reaktionsgemisch eingeleitete Amine oder Ammoniak. Die bevorzugte alkalisch wirkende Substanz ist Kaliumcarbonat, das bevorzugt als 10 %ige Lösung eingesetzt wird.

Die Menge an alkalisch wirkender Substanz ist nicht kritisch, solange der pH-Wert 7 des Reaktionsgemischs nicht überschritten wird. Andererseits aber versucht man, möglichst viel Huminat in Lösung zu bringen um eine möglichst hohe Ausbeute zu erzielen. Erfindungsgemäß werden daher zwei schnelle Methoden entwickelt, um die optimale Menge an alkalisch wirkender Substanz zu bestimmen:

Das huminstoffhaltige Produkt wird vor dem Suspendieren in entionisiertem Wasser vorgetrocknet (etwa durch 8 - 12 stündige Trocknung im Trockenofen bei 80 - 85 °C) und danach fein vermahlen und durchgemischt. Dadurch wird ein möglichst homogenes standardisiertes Einsatzprodukt erzeugt.

Mehrere 100 g Proben dieses Einsatzproduktes werden nun etwa 15 bis 20 h bei 120 - 140 °C scharf getrocknet und der verbleibende Rest als Trockenmasse bestimmt. Der Feststoffgehalt der Trockenmasse liegt je nach Art der eingesetzten huminstoffhaltigen Materials im Bereich von 50 bis 75 % des standardisierten Einsatzproduktes. Erfindungsgemäß werden pro 100 g Trockenmasse 50 - 100 mmol einer alkalisch wirkenden Substanz eingesetzt.

Die zweite Möglichkeit besteht in einer Titration einer Probe des standardisierten Einsatzproduktes. Bestimmt wird dabei die Alkalimenge, die notwendig ist, um den letzten Umschlagspunkt vor pH 7 zu erreichen. Diese ist die im Herstellungsverfahren einzusetzende Alkalimenge. Das entsprechende Vielfache dieser Alkalimenge wird unter genauester pH-Kontrolle zu einer Aufschlämmung des standardisierten Einsatzproduktes in entionisiertem Wasser zudosiert. Danach wird noch etwa 1/2 bis 3 h nachgerührt, bis sich ein konstanter pH-Wert einstellt. Dieser liegt bevorzugt im Bereich von 6,3 - 6,5. Nun wird etwa 1 bis 2 h sedimentiert und die überstehende, tiefbraune Lösung vorsichtig vom abgesetzten Schlamm getrennt.

Die so erhaltene, weitgehende feststofffreie Lösung wird in eine Zentrifuge bis 8.000 - 10.000 x g weiter von ungelösten Teilen befreit.

Danach wird die so geklärte Lösung einer Ultrafiltration unterworfen, bei der der Porenbereich der Filter im Bereich von 50.000 - 1.000.000 Dalton liegt.

Die erhaltene Lösung hat einen Gehalt an niedermolekularem Huminat von 2 - 10 Gew.-%. Sie kann direkt verwendet werden oder durch vorsichtiges Entfernen von Wasser, etwa mittels Gefriertrocknung zu einem Feststoff mit mindestens 4 % Restfeuchte getrocknet werden.

Dies ist ein weiterer Vorteil des erfindungsgemäßen Verfahrens, bei dem die niedermolekularen Huminate keinerlei Anteile mittelmolekularer Huminatstoffe enthalten.

Nach älteren Verfahren hergestellte niedermolekulare Huminate, die noch geringe Mengen mittelmolekularer Anteile enthalten, lassen sich lediglich bis zu höheren Konzentrationen aufkonzentrieren, erleiden jedoch bei weiterem Wasserentzug - auch mit den schonendsten Verfahren - eine irreversible Zerstörung.

Der erfindungsgemäß erhaltene Feststoff mit 4 % Restfeuchte bzw. eine konzentrierte wäßrige Lösung sind stabil.

Bei Stabilitätskontrollen ist nach 60 Tagen Wechselbelastung 56/4 °C im 12/12 h Rhythmus keine Veränderung der Parameter Gehalt, pH-Wert, Redoxspannung und Mikrodialysetest über die Zufallsschwankungen hinaus feststellbar.

Zu Bestimmung des ungefähren Molekulargewichts wird eine Probe der Lösung einer Elektrophorese unterworfen. Aus der elektrophoretischen Wanderungsgeschwindigkeit wird für die so hergestellten Huminate ein mittleres Molekulargewicht von 1.000 bei einem Streubereich von bis 1500 ermittelt.

Toxizitätsprüfungen ergeben, daß die erhaltenen niedermolekularen Huminate gering toxisch sind. Andererseits wurde gefunden, daß sie ebenfalls die bereits beobachtete Heilwirkung, insbesondere bei der Wundheilung zeigen.

Sie sind daher als Heilmittel insbesondere als Heilmittel in der Wundheilung geeignet.

Für viele Anwendungen ist jedoch eine wäßrige Lösung ungeeignet und man benötigt den Wirkstoff wasserfrei.

In einer älteren Anmeldung der Anmelderin (EP-A-283 573) (Patentanmeldung P 37 09 353.3) ist ein Verfahren zur Herstellung einer wasserfreien Applikationsform der niedermolekularen Huminate beschrieben, bei dem eine wäßrige Lösung dieser Huminate mit dem 0,2 - 5fachen Volumen eines anorganischen Trägermaterials vermischt und die erhaltene Mischung getrocknet wird.

Dieses Verfahren kann in das erfindungsgemäße Herstellungsverfahren integriert werden.

Als anorganisches Trägermaterial kann Aluminiumoxid, Titandioxid, Siliciumdioxid, insbesondere hochdisperse Kieselsäure oder Ton, insbesondere Montmorillonit oder Bentonit direkt mit der aus der Ultrafiltration gewonnenen Lösung angeteigt und die erhaltene Masse getrocknet werden.

Es hat sich als günstig erwiesen, 10 - 100 %, bezogen auf den Trockengehalt der Lösung, an anorganischem Trägermaterial mit der Lösung zu vermischen und die entstandene Suspension kontinuierlich an einem Dünnschichtverdampfer zu trocknen. Erhalten wird ein lagerstabiles, rieselfähiges Pulver, das alleine oder mit Hilfsstoffen als Puder, in Sprays oder wasserfreien Salben eingesetzt werden kann.

Darüberhinaus aber kann eine niedermolekulare Huminatfraktion die auf einen Gehalt von 4 bis 8 % Restfeuchte getrocknet wurde, auch durch handelsübliche Zucker und Stärken bzw. Stärkemodifikationen in Form von Verreibungen in eine stabile, rieselfähige Applikationsform gebracht werden. Bezogen auf die Wirkstoffeinwaage sind Verreibungen mit 20 bis 200 % Zucker bzw. Stärkegehalt sinnvoll. Erhalten wird ein lagerstabiles, rieselfähiges Pulver, das alleine oder mit weiteren Hilfsstoffen in entsprechenden Arzneiformen, bevorzugt als Puder, in Sprays, Salben oder Haftpasten eingesetzt werden kann.

Beispiel 1

20 kg Humusschlamm werden in einem Trockenofen 12 h lang bei 80 °C vorgetrocknet und anschließend in einer Mühle zu 1 - 3 mm großen Partikeln zermahlen und so homogenisiert und standardisiert.

Eine 1 g-Probe des standardisierten Humusschlamms wird in destilliertem Wasser aufgeschlämmt und mit 0,1 m NaOH titriert wobei die pH-Änderungen potentiometrisch gemessen werden. Bei 6,9 ml 0,1 m NaOH wird ein Umschlagspunkt im Bereich von pH 6 - 6,5 beobachtet.

Daher werden 0,69 mmol Lauge pro g als optimale Alkalimenge für dieses standardisierte huminstoffhaltige Produkt angesehen.

In einem Rührwerkbehälter werden 10 kg standardisierter Humusschlamm in 100 l destilliertem Wasser aufgeschlämmt. Unter Rühren werden 117 g gasförmiges $NH_3$ langsam in die Susupension eingeleitet. Der pH-Wert der Susupension, der während diesem Vorgang laufend kontrolliert wird, steigt von ursprünglich 3,9 stetig auf 6,3 an. Spätestens ab diesem Wert wird der Zustrom des Ammoniaks so gedrosselt, daß der pH-Wert im Bereich von 6,3 - 6,5 bleibt, d. h., daß die Menge der zugegebenen alkalisch wirkenden Substanz der entspricht, die im gleichen Zeitraum bei der Neutralisation der komplexen Huminstoffe verbraucht wird.

Nach der $NH_3$-Zugabe, die nach 70 min beendet ist, wird noch 1 h weitergerührt. Danach wird der Rührer abgestellt. Die suspendierten Feststoffe lagern sich als Schlamm am Boden des Behälters ab. Die überstehende braune Lösung wird abdekantiert und direkt in einem Separator bei 9.000 x g zentrifugiert. Die so erhaltene gereinigte Lösung wird einer Ultrafiltration mit einem Ultrafilter der Porenweite 100.000 - 250.000 D unterworfen. Erhalten wird eine dunkelbraune Lösung mit einem Gehalt an 2,1 % einer Ammoniumhuminatfraktion mit einem mittleren Molekulargewicht von 1000 und einem Streubereich von 300 bis 1500.

Beispiel 2

20 kg Heilerde werden in einem Trockenofen 12 h lang bei 80 °C vorgetrocknet und anschließend in einer Mühle zu 1 - 3 mm großen Partikeln zermahlen und so homogenisiert und standardisiert.

Eine 100 g Probe der standardisierten Heilerde wird während 16 h bei 130 °C scharf getrocknet und anschließend der Gewichtsverlust bestimmt. Daraus wurde ein Feststoffgehalt von 69 Gew.-% ermittelt.

In einem Rührwerksbehälter werden 10 kg standardisierte Heilerde in 100 l destilliertem Wasser aufgeschlämmt wobei sich ein pH-Wert von 3,5 einstellt. Unter Rühren und unter ständiger Kontrolle des pH-Wertes der Suspension werden 7.630 g 10%iger $K_2CO_3$-Lösung (entsprechend 5.520 mmol $K_2CO_3$ oder entsprechend 80 mmol $K_2CO_3$/100 g scharf getrockneter Heilerde) so langsam zur Suspension dosiert, daß ein pH-Wert von 7 nicht überschritten wird.

Nach der $K_2CO_3$-Zugabe, die nach 60 min beendet ist, wird noch 1 h weitergerührt, wobei sich ein konstant bleibender pH-Wert von 6,5 einstellt. Danach wird der Rührer abgestellt. Die suspendierten Feststoffe lagern sich als Schlamm am Boden des Behälters ab. Die überstehende braune Lösung wird abdekantiert und direkt in einem Separator bei 9.000 x g zentrifugiert. Die so erhaltene gereinigte Lösung wird einer Ultrafiltration mit einem Ultrafilter der Porenweite 100.000 - 250.000 D unterworfen. Erhalten wird eine dunkelbraune Lösung mit einem Gehalt an 3,2 % einer Kaliumhuminatfraktion mit einem mittleren Molekulargewicht von 1000 und einem Streubereich von 300 bis 1500.

Beispiel 3

In 5 l der in Beispiel 2 erhaltenen Kaliumhuminatlösung werden 32 g Titandioxidpulver suspendiert und die erhaltene Suspension wird kontinuierlich mit Hilfe eines Dünnschichtverdampfers zur Trockne eingedampft. Erhalten wird ein graues, rieselfähiges Produkt, das bis 130 °C beständig ist und aus dem bei Kontakt mit Wasser der Wirkstoff wieder herausgelöst wird.

Beispiel 4

Mit den in den Beispielen 1 und 2 erhaltenen Lösungen (1) und (2) werden die nachfolgend beschriebenen Untersuchungen durchgeführt und die aufgeführten Ergebnisse erhalten.

Toxität:

Bei Injektionen der 1%igen Lösung an Versuchstieren (Mäuse) werden folgende Werte der $LD_{50}$ erhalten.

|          | Lösung (1)     | Lösung (2)     |
|----------|----------------|----------------|
| s.c.     | 1292 (mg/kg)   | 1136 (mg/kg)   |
| i.p.     | 853            | 784            |
| i.V.     | 676            | 722            |

Stabilität:

Bei der Stabilitätskontrolle ist nach 60 Tagen Wechselbelastung 56/4 °C im 12/12 h Rhythmus keine Veränderung der Parameter Gehalt, pH-Wert, Redoxspannung und Mikrodialysetest über die Zufallsschwankungen hinaus feststellbar.

Heilwirkung:
Fibroblastentest

Eine nach Trypsinbehandlung in Suspension gebrachte Kultur von L - Zellen (Mäusefibroblasten) wird mit 50 ppm niedermolekularem Alkalihuminat (Lösungen aus Beispiel 1 und 2) versetzt.
Die Kultur wird bei 37 °C unter Verwendung eines handelsüblichen Nährmediums 48 h lang gebrütet.
Parallel hierzu wird als Vergleichsversuch eine analoge Kultur ohne Alkalihuminat gleichermaßen bebrütet. Danach wird die Anzahl der lebenden Zellen in beiden Kulturen bestimmt. In den mit niedermolekularen Alkalihuminaten versetzten Kulturen liegt die Zahl der lebenden Zellen um 30 % höher als in der Vergleichskultur.

Wundheilung:

An 2 x 10 haarlosen Mäusen werden mit einem Mikrodermatom oberflächliche Wunden, die nur die obersten Epithelschichten erfaßten, in einer Größe von etwa 50 mm$^2$ beigebracht. Bei je zehn dieser Mäuse wird die Wunde mit jeweils einer 1 %igen Lösung (aus Beispiel 1 und 2) einmal benetzt. Die anderen Mäuse bleiben unbehandelt.
Während des Beobachtungszeitraumes von 7 d läßt sich folgendes beobachten: Gegenüber den unbehandelten Mäusen nimmt bei den behandelten Versuchstieren
die Wundflächen rascher ab,
die Wunde trocknet früher ab,
die Granulation setzt früher ein und
die Wunde reinigt sich früher.
Insgesamt ist die Abheilung 2 - 3 d früher als bei den Kontrolltieren zu beobachten.

**Patentansprüche**

1. Verfahren zur Herstellung einer niedermolekularen Huminatfraktion, **dadurch gekennzeichnet,** daß zu einer wäßrigen Suspension huminstoffhaltiger Produkte unter Rühren eine alkalisch wirkende Substanz so zudosiert wird, daß der pH-Wert 7 nicht überschritten wird, die Suspension bis zur Konstanz des pH-Wertes weitergerührt wird, danach sedimentiert und die weitgehend feststofffreie Lösung abgetrennt und danach zentrifugiert wird und die dabei erhaltene geklärte Lösung einer Ultrafiltration unterworfen wird.

2. Verfahren nach Anspruch 1, **dadurch gekennzeichnet,** daß das huminstoffhaltige Produkt vor dem Suspendieren vorgetrocknet wird.

3. Verfahren nach den Ansprüchen 1 und 2, **dadurch gekennzeichnet,** daß als alkalisch wirkende Substanz Alkalihydroxid, Alkalicarbonat oder Ammoniak verwendet wird.

4. Verfahren nach den Ansprüchen 1 bis 3, **dadurch gekennzeichnet,** daß als alkalisch wirkende Substanz Kaliumcarbonat verwendet wird.

**5.** Verfahren nach den Ansprüchen 1 bis 4, **dadurch gekennzeichnet,** daß ein konstanter pH-Wert im Bereich von pH 6,3 - 6,5 eingestellt wird.

**6.** Verfahren nach den Ansprüchen 1 bis 4, **dadurch gekennzeichnet,** daß die Menge der zudosierten alkalisch wirkenden Substanz im Bereich von 50 bis 100 mmol der alkalisch wirkenden Substanz / 100 g Trockenmasse des huminstoffhaltigen Produkts liegt.

**7.** Verfahren nach den Ansprüchen 1 bis 6, **dadurch gekennzeichnet,** daß die abgetrennte, weitgehend feststofffreie Lösung bei 8.000 - 10.000 x g zentrifugiert wird.

**8.** Verfahren nach den Ansprüchen 1 bis 7, **dadurch gekennzeichnet,** daß die Ultrafiltration mit einem Porenbereich von 50.000 bis 1.000.000 Dalton durchgeführt wird.

**9.** Verfahren nach den Ansprüchen 1 bis 8, **dadurch gekennzeichnet,** daß die aus der Ultrafiltration erhaltene Lösung mit 10 - 100 Gew.-%, bezogen auf den Feststoffgehalt der Lösung eines anorganischen Trägermaterials versetzt und zu einem rieselfähigen Produkt getrocknet wird.

**10.** Verfahren nach Anspruch 9, **dadurch gekennzeichnet,** daß als anorganisches Trägermaterial Titandioxidpulver verwendet wird.

**11.** Verfahren nach den Ansprüchen 1 bis 8, **dadurch gekennzeichnet,** daß die aus der Ultrafiltration erhaltene Lösung zu einem festen Produkt mit einer Restfeuchte von 4 bis 8 % getrocknet und danach in an sich bekannter Weise mit einem Zucker, Stärke oder Stärkemodifikationen zu einem rieselfähigen Produkt verrieben wird.

**12.** Verwendung der nach den Ansprüchen 9 oder 11 hergestellten rieselfähigen Produkte in entsprechenden Arzneiformen.

**13.** Verwendung der nach den Ansprüchen 9 oder 11 hergestellten rieselfähigen Produkte als Puder, in Sprays, Salben oder Haftpasten.

## Claims

**1.** A method of producing a low-molecular huminate fraction, characterised in that a substance having an alkaline action is added in doses to an aqueous suspension of products containing humic matter whilst agitating, in such a manner that the pH value does not exceed 7, the suspension is agitated further until a constant pH value is achieved, subsequently settles out, and the solution, which is substantially free of solid matter, is separated and subsequently centrifuged and the clarified solution obtained is subjected to ultrafiltration.

**2.** A method according to Claim 1, characterised in that the product containing humic matter is predried before suspension.

**3.** A method according to Claims 1 and 2, characterised in that alkali hydroxide, alkali carbonate or ammonia is used as the alkaline-acting substance.

**4.** A method according to Claims 1 to 3, characterised in that potassium carbonate is used as the alkaline-acting substance.

**5.** A method according to Claims 1 to 4, characterised in that a constant pH value is located in the range of pH 6. 3 - 6. 5.

**6.** A method according to Claims 1 to 4, characterised in that the quantity of the alkaline-acting substance added in doses is in the range of 50 to 100 mmol of alkaline-acting substance per 100 g dry mass of the product containing humic matter.

**7.** A method according to Claims 1 to 6, characterised in that the separated solution, which is substantially free of solid matter, is centrifuged at 8,000 - 10,000 x g.

**8.** A method according to Claims 1 to 7, characterised in that the ultrafiltration is carried out with a pore range of 50,000 to 1,000,000 daltons.

**9.** A method according to Claims 1 to 8, characterised in that the solution obtained by ultrafiltration is mixed with 10 - 100 % by weight - relative to the solid matter content of the solution - of an inorganic carrier material and is dried to form a flowable product.

**10.** A method according to Claim 9, characterised in that titanium dioxide powder is used as the inorganic carrier material.

**11.** A method according to Claims 1 to 8, characterised in that the solution obtained by ultrafiltration is dried to form a solid product with a residual moisture of 4 to 8 % and subsequently triturated in a manner known per se with a sugar, starch or modified forms of starch to form a flowable product.

**12.** A use of the flowable products produced according to Claims 9 or 11 in appropriate medicinal forms.

**13.** A use of the flowable products produced according to Claims 9 or 11 as powders, in sprays, ointments or adhesive pastes.

**Revendications**

**1.** Procédé pour la préparation d'une fraction d'humates de bas poids moléculaire, caractérisé en ce qu'à une suspension aqueuse de produits contenant des substances humiques, on ajoute en quantité dosée, sous agitation, une substance à effet alcalin de telle manière que la valeur de pH 7 ne soit pas dépassée, on poursuit l'agitation de la suspension jusqu'à ce que la valeur de pH soit constante, après quoi on laisse sédimenter la suspension, on sépare et centrifuge ensuite la solution dans une large mesure exempte de matières solides et on soumet la solution clarifiée ainsi obtenue à une ultrafiltration.

**2.** Procédé selon la revendication 1, caractérisé en ce que le produit contenant des substances humiques est préséché avant sa mise en suspension.

**3.** Procédé selon les revendications 1 et 2, caractérisé en ce qu'on utilise un hydroxyde alcalin, un carbonate alcalin ou de l'ammoniaque en tant que substance à effet alcalin.

**4.** Procédé selon les revendications 1 à 3, caractérisé en ce qu'on utilise du carbonate de potassium en tant que substance à effet alcalin.

**5.** Procédé selon les revendications 1 à 4, caractérisé en ce qu'on ajuste une valeur de pH constante dans le domaine de pH 6,3 - 6,5.

**6.** Procédé selon les revendications 1 à 4, caractérisé en ce que la quantité de substance à effet alcalin ajoutée de façon dosée se situe dans le domaine de 50 à 100 mmoles de la substance à effet alcalin/100 g de masse sèche du produit contenant des substances humiques.

**7.** Procédé selon les revendications 1 à 6, caractérisé en ce que la solution séparée, dans une large mesure exemple de matières solides, est soumise à une centrifugation à 8.000 - 10.000 x g.

**8.** Procédé selon les revendications 1 à 7, caractérisé en ce que l'ultrafiltration est effectuée dans un domaine de pores de 50.000 à 1.000.000 Dalton.

**9.** Procédé selon les revendications 1 à 8, caractérisé en ce que la solution obtenue à partir de l'ultrafiltration est additionnée de 10 - 100% en poids, rapportés au taux de solides de la solution, d'un support inorganique et est séchée en donnant un produit à écoulement libre.

**10.** Procédé selon la revendication 9, caractérisé en ce qu'on utilise de la poudre de dioxyde de titane comme support inorganique.

7

**11.** Procédé selon les revendications 1 à 8, caractérisée en ce que la solution obtenue à partir de l'ultrafiltration est séchée jusqu'à obtention d'un produit solide avec une humidité relative de 4 à 8% et qu'il est ensuite trituré de façon connue en soi avec un sucre, de l'amidon ou des amidons modifiés en vue de l'obtention d'un produit à écoulement libre.

**12.** Utilisation des produits à écoulement libre préparés selon la revendication 9 ou 11 sous des formes médicamenteuses correspondantes.

**13.** Utilisation des produits à écoulement libre préparés selon la revendication 9 ou 11 sous forme de poudres, dans des sprays, des pommades ou des pâtes adhésives.